# DEMANDE DE BREVET EUROPEEN

(11) **EP 4 209 200 A1**
(43) Date de publication de la demande: **12.07.2023**
(21) Numéro de dépôt: 23150770.8
(22) Date de dépôt: 09.01.2023
(51) Int. Cl.: A61F 9/00, G02C 7/04

(54) **STRUCTURES ANNULAIRES CONSTITUÉES DE MEMBRANE AMNIOTIQUE RÉTICULÉE**

(30) Priorité: 08.01.2022 FR 2200127; 29.06.2022 FR 2206571
(71) Demandeur: TBF Genie Tissulaire (TBF), 69780 Mions (FR)
(72) Inventeur: BARNOUIN, Laurence, 38460 VENERIEU (FR)
(74) Mandataire: Tripoz, Inès

(57) **Abrégé**

L'invention concerne une structure annulaire constituée de membrane amniotique ayant subi une étape de réticulation. Selon les modes de réalisation ladite structure annulaire peut être obtenue par enroulement de ladite membrane amniotique autour d'un support de type filaire avant l'étape de réticulation. L'invention concerne également un dispositif, utilisable comme lentille, comprenant : une membrane amniotique centrale et une structure annulaire selon l'invention, solidaire de ladite membrane centrale et disposée à la périphérie de celle-ci. L'invention concerne également le procédé d'obtention de telles structures.

## Description

La présente invention concerne le domaine dispositifs médicaux et plus particulièrement ceux obtenus à partir de membranes amniotiques. Dans un mode de réalisation ces membranes amniotiques sont utilisées dans le traitement des surfaces oculaires pour la fabrication de « lentilles pansement » et pour la réalisation de structures annulaires qui peuvent être utilisées comme support dans des dispositifs médicaux.

La membrane chorioamniotique, qui sépare le foetus de l'endomètre de la mère chez les mammifères, inclut la membrane amniotique, ou amnios, et la membrane choriale, ou chorion. Ces deux membranes sont reliées par une membrane tissulaire spongieuse, aussi appelée couche spongieuse, constituée entre autres de collagène et de protéoglycanes, la membrane tissulaire spongieuse comportant des ponts protéiques, attachés de part et d'autre à l'amnios d'une part, et au chorion, d'autre part.

La membrane amniotique est la couche la plus interne de la membrane chorioamniotique. Elle a pour rôle de protéger le foetus et de maintenir autour de lui le liquide amniotique. Cette membrane amniotique est un tissu très fin, transparent qui comporte plusieurs couches, à savoir une couche épithéliale, une membrane basale, une couche compacte et une couche fibroblastique.

Non vascularisée et non innervée, la membrane amniotique est riche en collagène et en divers facteurs de croissance, et présente des propriétés participant au processus de cicatrisation.

La membrane amniotique, obtenue à partir du placenta après un accouchement, est un tissu utilisé depuis plus de cent ans dans le traitement des brûlures et des blessures. En effet, dès 1910, Davies utilisait des membranes foetales tant sur des brûlures que sur des tissus ulcérés. Trelford et Trelford-Sauder rapportent qu'en 1935 des auteurs ont publié des applications cliniques de la membrane amniotique en vaginoplastie, reconstruction conjonctivale, traitement des brûlures ou des blessures, et traitements relatifs à l'adhérence intra-abdominale. Trelford *et al.* rapportent aussi qu'en 1952 Douglas a utilisé de l'amnios pour traiter des blessures étendues. Pour la première fois, il fut indiqué que la couche stromale de la membrane amniotique, comprenant la couche compacte et la couche fibroblastique, permettait une adhérence plus importante de la greffe, et donc de son efficacité. En 1972, les travaux de Trelford *et al.* ont confirmé ce fait. Gindraux *et al.* rapportent qu'à partir de 1972, et surtout depuis sa redécouverte en 1995, d'autres auteurs ont confirmé toutes les applications cliniques présentées précédemment, et ont également signalé de nouvelles indications telles que le tractus génito-urinaire, l'estomac, le larynx, la cavité orale, la tête et le cou, que ce soit dans des essais cliniques ou des rapports de cas.

Dans le domaine de l'ophtalmologie, c'est en 1940 que De Roetth a utilisé pour la première fois une membrane amniotique en ophtalmologie reconstructive pour traiter des altérations conjonctivales de patients.

Ce type de produit biologique est particulièrement apprécié dans le traitement des maladies inflammatoires de la cornée, telles que les ulcères cornéens ; ou dans le traitement de destructions de la surface de la cornée, induites par exemples par des brûlures à l'acide, le syndrome de Stevens-Johnson ou le syndrome de Lyell.

La greffe de membrane amniotique permet au niveau local la fourniture d'une structure, avasculaire et acellulaire, servant de membrane basale de support à l'épithélium cornéen. En conséquence, cette technique évite les phénomènes de rejet et d'inflammation locale, tout en facilitant la cicatrisation épithéliale cornéenne.

Outre la greffe, la membrane amniotique peut également être utilisée comme pansement, ou patch, lorsque sa propre surface épithéliale est suturée de sorte à être en contact avec la surface de l'oeil. Dans ce cas, la membrane délivre les facteurs biologiques dont elle est riche vers la cornée du patient afin de promouvoir la cicatrisation de celle-ci.

Les membranes amniotiques sont particulièrement riches en facteurs de croissance de type EGF (Epidermal Growth Factor), TGF (Transforming Growth Factor) et KGF (Keratinocyte Growth Factor), ainsi qu'en acide hyaluronique. Ces facteurs de croissance et l'acide hyaluronique sont particulièrement adaptés pour promouvoir les processus biologiques de cicatrisation, notamment la cicatrisation de l'épithélium cornéen.

Toutefois, la manipulation et la mise en place d'un dispositif médical constitué de fragments de membrane amniotique que ce soit comme greffe ou comme pansement, sont difficiles car la membrane est fragile et requiert parfois l'utilisation d'un support pour être mise en place, voire de suture sur l'épithélium recevant la greffe.

Il existe donc un besoin pour un dispositif médical permettant l'utilisation d'une membrane amniotique dans le traitement des lésions de l'épithélium et plus particulièrement l'épithélium cornéen, sans qu'il soit nécessaire de procéder à la suture de la membrane pour la maintenir en place.

Plusieurs supports pour membranes amniotiques, destinés à être utilisés dans le traitement des lésions de l'épithélium cornéen, sans qu'il soit nécessaire de procéder à la suture desdits supports, ont été proposés.

Dans le document WO2003077794 au nom de TISSUETECH sont décrits différents dispositifs où une membrane amniotique est fixée sur un anneau support de différentes manières. La membrane peut être fixée sur l'anneau support à l'aide d'une colle, s'enrouler autour de l'anneau support, être insérée dans l'anneau support, être clipsée à l'anneau support à l'aide d'un second anneau.

Dans le document US2009/143792 est décrit un dispositif où une membrane est fixée entre deux anneaux disposés de manière concentriques l'un par rapport à l'autre. L'anneau interne est de structure rigide, l'anneau externe est de structure élastique. Les deux anneaux associés présentent une surface interne commune en forme de cône tronqué de sorte à s'adapter à la convexité de l'oeil. La membrane amniotique étant clipsée entre 2 anneaux, une contrainte mécanique est exercée sur celle-ci pouvant conduire à des risques de rupture au montage et à l'usage.

La demande WO2016138025 au nom de TISSUETECH divulgue un dispositif de lentille comprenant un support de membrane sous forme de structure tubulaire flexible pouvant être en différents matériaux incluant des tissus biologiques. Ce dispositif requiert l'utilisation de moyens flexibles pour fixer la membrane sur le support.

Le problème posé par ces dispositifs est qu'ils ont tous recourt à des moyens de fixation de la membrane amniotique à leur support. Lors de cette étape de fixation, mais également à l'usage, des contraintes mécaniques sont appliquées à la membrane amniotique qui est un matériau fragile, pouvant ainsi entrainer une rupture ou une dégradation de la membrane. La manipulation lors de l'assemblage du dispositif et son utilisation se doit donc d'être très minutieuse et peut s'avérer contraignante.

On connait de WO2020245324 au nom de la demanderesse un dispositif qui ne requiert pas de moyens de fixation de la membrane au support, en ce qu'il s'agit du support qui est appliqué sur la membrane sous forme de 2 couches de gel formant une structure annulaire entourant le contour extérieur de la membrane, chacune des 2 couches étant appliquée sur une face différente du contour externe de la membrane. Une étape ultérieure de solidification du gel permettra le maintien de la membrane entre les 2 couches de gel formant un support rigide pour la membrane. De ce fait aucune contrainte mécanique n'est appliquée à la membrane lors de l'assemblage du dispositif, ni lors de son utilisation. Cependant, le matériau sous forme de gel ajouté pour former la structure annulaire entourant le contour externe de la membrane est susceptible de causer des réactions inflammatoires lors de l'application sur le patient.

Cependant toutes les fixations qui ne présentent pas de risque de dégradation de la membrane peuvent se révéler insuffisamment solides pour résister aux contraintes subies lors de la pose du dispositif et/ou lors de l'étape de réhydratation.

Le dispositif selon la présente invention permet de résoudre le problème posé par les dispositifs de l'art antérieur en ce que la structure annulaire est elle-même constituée de membrane amniotique (2) ayant subi une étape de réticulation.

On connait de US2018193133A1, un revêtement thérapeutique pour le traitement des défauts épithéliaux de la cornée comprenant une couche de matériaux thérapeutiques pouvant comprendre un tissu à base de collagène stabilisé et/ou solidifié par trempage dans un photosensibilisateur et exposé à la lumière pour induire une réticulation photocatalysée.

Dans cette demande le matériau thérapeutique réticulé est retrouvé au centre du dispositif sous forme de lentille pansement.

On connait également WO2008/060377A2, une composition comprenant des composants solubles notamment issus de membrane amniotique et pouvant comprendre de la riboflavine en tant qu'agent réticulant, ladite composition pouvant être sous la forme de lentille pansement.

On connait enfin de WO2006/002128A1, une membrane amniotique isolée traitée avec un composant modificateur de consistance, ce composant pouvant être un agent réticulant, pour augmenter la rigidité de la membrane. Ladite membrane peut ensuite être moulée ou façonnée pour former un dispositif, notamment une lentille.

Ainsi, les demandes US2018193133A1, WO2008/060377A2 et WO2006/002128A1 ne divulguent pas de structures annulaires constituées de membrane amniotique ayant subi une étape de réticulation.

On connait également de RU2652078C1 un procédé de traitement des ulcères de la cornée comprenant les étapes séquentielles de réticulation de la cornée suivie de la suture de membrane amniotique sur la zone désépithélialisée. Le procédé de traitement est donc différent de la présente invention en ce que dans le procédé selon RU2652078, c'est la cornée qui est réticulée et non la membrane amniotique.

A la différence de US2018193133A1, WO2008/060377A2 et WO2006/002128A1, la structure annulaire réticulée selon la présente invention permet donc de disposer d'une structure externe rigide moins fragile et plus simple à mettre en place sur le patient, ayant une excellente biocompatibilité.

La structure inédite mise au point par la demanderesse permet de manière tout à fait surprenante de conserver une partie centrale libre pour la mise en place d'une membrane amniotique non réticulée, qui conserve tous les avantages structurels natifs de celle-ci, notamment sa flexibilité intrinsèque, lui permettant de se conformer parfaitement à la courbure de la cornée.

Ce dispositif présente notamment l'avantage de disposer d'une structure annulaire sans nécessiter d'addition de matériau autre que de la membrane amniotique.

Selon les modes de réalisation ladite structure annulaire peut être obtenue par enroulement de ladite membrane amniotique (2) autour d'un support de type filaire (1) avant l'étape de réticulation.

La [Fig. 1] illustre une coupe transversale d'un mode de réalisation d'une structure annulaire selon l'invention utilisable comme lentille. La zone délimitée A représente la zone soumise à la réticulation.

La [Fig. 2] illustre un mode de réalisation d'une structure cylindrique.

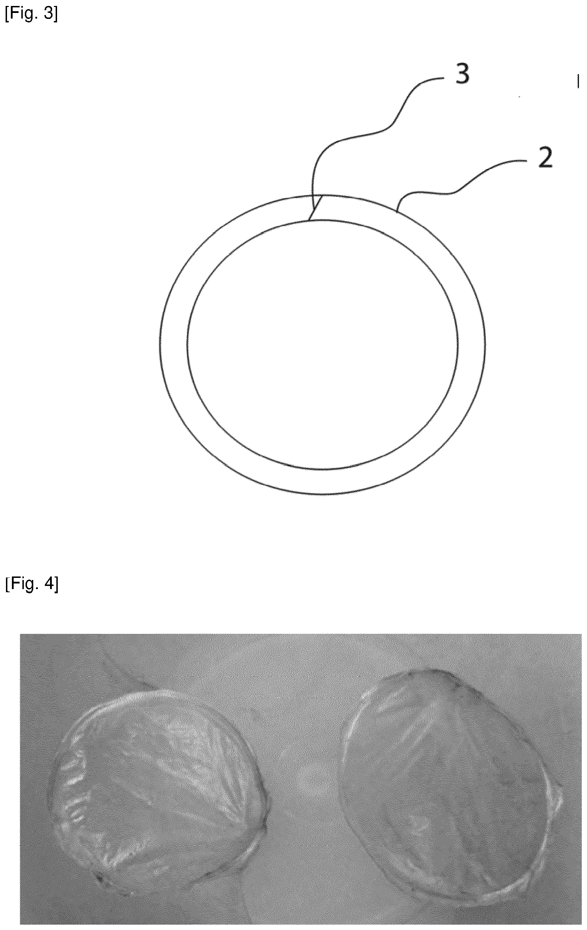

La [Fig. 3] illustre un autre mode de réalisation de l'invention, à savoir un anneau obtenu à partir d'une structure cylindrique comprenant une suture (3).

La [Fig. 4] est une photographie de deux dispositifs utilisables comme lentille, comprenant une structure annulaire constituée de membrane amniotique ayant subi une étape de réticulation entourant une membrane amniotique centrale selon l'invention.

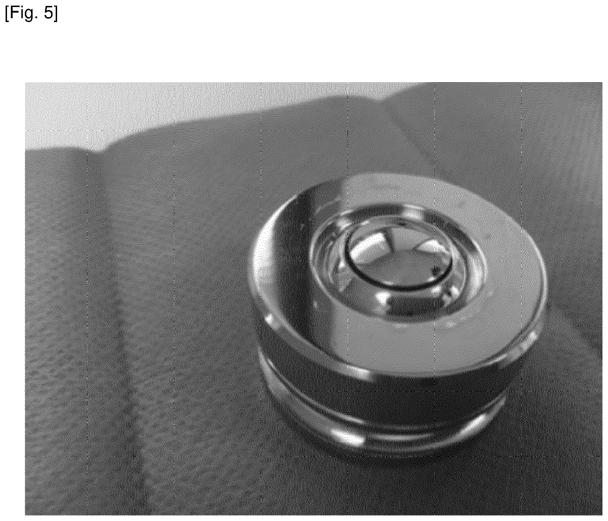

La [Fig. 5] est une photographie d'un moule transparent à la lumière UV et comportant une rigole dans laquelle est introduite l'agent photosensibilisateur.

La présente invention concerne une structure annulaire constituée de membrane amniotique (2) ayant subi une étape de réticulation.

Elle concerne également une structure cylindrique constituée de membrane amniotique (2) ayant subi une étape de réticulation.

Au sens de la présente invention, on entend par "réticulation" la formation de liaisons additionnelles qui est une méthode bien connue et utilisée pour la stabilisation de polymères dans l'industrie permettant la formation de liaisons transversales transformant un polymère linéaire en polymère tridimensionnel. La réticulation peut être obtenue par toutes les méthodes classiquement utilisées par l'Homme du métier.

L'étape de réticulation présente l'avantage de conserver l'enroulement de la forme annulaire ou de la forme cylindrique sans montage compliqué ni apport de matériau supplémentaire et ce, même si la structure annulaire ou cylindrique est lyophilisée puis réhydratée.

Dans un mode de réalisation, l'étape de réticulation selon l'invention est réalisée par glycation non enzymatique, irradiation à la lumière UV avec ou sans un agent photosensibilisateur et/ou par réaction aldéhydique.

Dans un mode de réalisation, l'étape de réticulation selon l'invention est réalisée par irradiation à la lumière UV avec un agent photosensibilisateur.

Dans un mode de réalisation, l'agent photosensibilisateur est la riboflavine ou un de ses sels.

Dans un mode de réalisation, le sel de riboflavine est le sel de sodium phosphaté de riboflavine.

Dans un mode de réalisation, l'étape de réticulation comprend la réticulation du collagène présent dans la membrane amniotique.

Dans un mode de réalisation, dans la membrane amniotique enroulée sur elle-même, et éventuellement autour d'un fil, l'étape de réticulation comprend la réticulation d'au moins une molécule de collagène d'une face de la membrane amniotique avec au moins une molécule de collagène d'au moins une autre face de la membrane amniotique.

De cette façon, les différentes épaisseurs de membrane enroulée sur elle-même sont fixées les unes aux autres permettant ainsi de conserver l'enroulement.

Dans un mode de réalisation, l'étape de réticulation selon l'invention est réalisée dans un moule transparent à la lumière UV et comportant une rigole dans laquelle est introduite l'agent photosensibilisateur.

Dans un mode de réalisation, la structure annulaire constituée de membrane amniotique (2), est de forme sensiblement circulaire.

Dans un mode de réalisation, la structure annulaire constituée de membrane amniotique (2), est de forme sensiblement ovale ou sous forme d'ellipse.

Dans un mode de réalisation, la structure annulaire selon l'invention est constituée de la membrane amniotique (2) enroulée sur elle-même et/ou autour d'un support de type filaire (1).

Dans un mode de réalisation, la structure annulaire selon l'invention consiste en de la membrane amniotique (2) enroulée sur elle-même et/ou autour d'un support de type filaire (1).

Dans un mode de réalisation, la structure annulaire selon l'invention consiste en de la membrane amniotique (2) enroulée sur elle-même.

Dans un mode de réalisation, la structure annulaire selon l'invention ne comprend pas de résines ou de gels.

Dans un mode de réalisation, la structure annulaire selon l'invention ne comprend pas de gels biocompatibles de polysaccharides, de gels de polymères de galactoses ou des gels de tissus biologiques, notamment de gelée de Wharton.

Dans un mode de réalisation, la structure annulaire ou la structure cylindrique selon l'invention comprend en outre un support de type filaire (1).

Dans un mode de réalisation, la structure annulaire selon l'invention possède une épaisseur comprise entre 1 et 4 mm.

Lorsque la structure annulaire selon l'invention est circulaire elle est définie par son diamètre lorsqu'elle est de forme elliptique ou ovale elle sera définie par un diamètre équivalent.

Dans un mode de réalisation, la structure annulaire selon l'invention possède un diamètre compris entre 1 et 3 cm.

Dans un mode de réalisation, la structure annulaire selon l'invention possède un diamètre compris entre 2 et 2,5 cm.

Dans un mode de réalisation, la structure cylindrique selon l'invention possède une épaisseur comprise entre 1 et 4 mm.

Dans un mode de réalisation, le support de type filaire selon l'invention est en matériau biocompatible.

Dans un mode de réalisation, le support de type filaire selon l'invention possède un diamètre inférieur ou égal à 3 mm

Dans un mode de réalisation, le support de type filaire selon l'invention possède un diamètre de 0,001 mm à 3 mm.

Dans un mode de réalisation, le support de type filaire selon l'invention possède un diamètre de 0,1 mm à 0,9 mm.

Dans un mode de réalisation, le support de type filaire selon l'invention est un fil de suture.

Dans un mode de réalisation, le fil de suture est choisi dans le groupe des fils en nylon, en polybutester, en polypropylène et/ou en soie.

Dans un mode de réalisation, le fil de suture est un fil de suture résorbable.

Dans un mode de réalisation, le fil de suture de résorbable est choisi dans le groupe des fils en glycolide et lactide, en polyglytone, en poliglecarpone, en polyglactin, en lactomer, en acide polyglycolique, en glycomer, en polyglyconate ou en polydioxanone.

L'utilisation d'un fil de suture présente l'avantage de disposer d'un matériau biocompatible et pouvant être retiré facilement de la structure annulaire ou cylindrique selon l'invention avant l'application sur le patient, sans endommager ladite structure annulaire ou cylindrique. L'utilisation d'un fil de suture résorbable permet d'utiliser directement la structure annulaire ou cylindrique selon l'invention sans avoir à le retirer de la structure annulaire sans risque pour le patient.

La présente invention concerne également un dispositif, utilisable comme lentille, comprenant :
- une membrane amniotique centrale ;
- une structure annulaire selon l'invention, solidaire de ladite membrane amniotique centrale et disposée à la périphérie de celle-ci.

Au sens de la présente invention, on entend par « lentille » une lentille biologique dite lentille pansement ou lentille thérapeutique.

Dans un mode de réalisation, le dispositif est une lentille.

Dans un mode de réalisation, le dispositif est une lentille pansement.

Selon la présente invention, la membrane amniotique centrale est la partie du dispositif utilisable comme lentille selon l'invention constituée de la membrane amniotique non enroulée.

Selon la présente invention, la membrane amniotique centrale peut être alternativement constituée de la membrane amniotique (2) dont est issue la structure annulaire selon l'invention ou constituée d'une membrane amniotique indépendante.

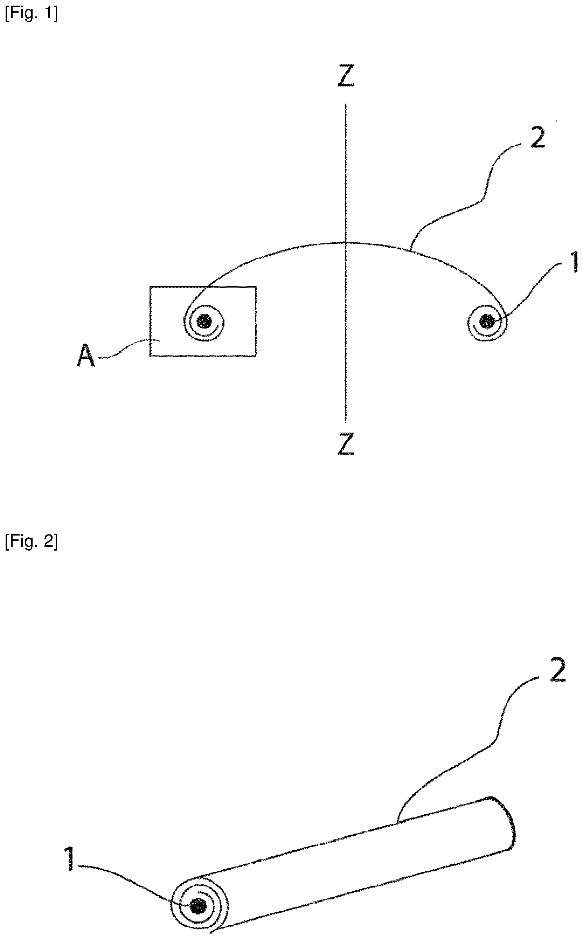

Dans un mode de réalisation, la membrane amniotique centrale est constituée de la membrane amniotique (2) et la structure annulaire selon l'invention est formée par enroulement de la périphérie de la membrane amniotique (2) sur elle-même et/ou autour d'un support de type filaire, tel qu'illustré en figure 1.

Dans un mode de réalisation, la membrane amniotique indépendante est issue du même organisme donneur que celui de la structure annulaire selon l'invention.

Dans un mode de réalisation, la membrane amniotique indépendante est issue d'un organisme donneur différent de celui de la structure annulaire selon l'invention.

Dans un mode de réalisation, le dispositif utilisable comme lentille selon l'invention est de forme sensiblement circulaire.

Dans un mode de réalisation, le dispositif utilisable comme lentille selon l'invention est de forme sensiblement ovale ou sous forme d'ellipse.

Dans un mode de réalisation, la membrane amniotique centrale du dispositif utilisable comme lentille selon l'invention n'est pas réticulée.

Dans un mode de réalisation, seule la structure annulaire selon l'invention est réticulée.

Le fait de ne pas réticuler la membrane amniotique centrale du dispositif utilisable comme lentille selon l'invention présente l'avantage de conserver sa structure physiologique et sa flexibilité intrinsèque.

Lorsque la présente invention concerne un dispositif de lentille biologique ou lentille pansement à des fins d'applications médicales, par exemple dans le traitement des surfaces oculaires, on fera référence à un dispositif selon l'invention positionné sur l'oeil d'un individu étant un être humain ou un mammifère. Dans le cas d'une application vétérinaire, le dispositif sera adapté notamment en fonction de la taille et la forme de l'oeil de l'animal.

Au sens de la présente invention, par « membrane amniotique », on entend l'enveloppe tissulaire qui se développe autour de l'embryon, puis du foetus, chez le mammifère durant la grossesse. Elle a pour rôle de protéger l'organisme en développement, en maintenant autour de lui le liquide amniotique. Elle s'accole à la deuxième membrane qui est le chorion. La membrane amniotique inclut les souscouches physiologiques suivantes : la couche cellulaire épithéliale, la membrane basale, la couche compacte, la couche fibroblastique, la couche spongieuse.

La membrane amniotique est souple. Cette propriété est avantageusement utilisée par la présente invention. En effet, selon l'invention une membrane amniotique (2) ou la membrane amniotique indépendante est prédécoupée selon une forme prédéfinie, par exemple sensiblement circulaire, ovale, elliptique ou rectangulaire.

Grâce à sa flexibilité intrinsèque, la membrane amniotique (2) peut être enroulée sur elle-même et/ou sur un support de type filaire (1).

Grâce à sa flexibilité intrinsèque, la membrane amniotique (2) permet au dispositif de structure cylindrique après enroulement d'être mis en une forme annulaire après l'étape de suture (3).

Grâce à la flexibilité intrinsèque de la membrane amniotique, la membrane amniotique (2) ou la membrane amniotique indépendante peuvent présenter une forme convexe lorsque l'invention concerne un dispositif utilisable comme lentille, une fois le dispositif utilisable comme lentille selon l'invention positionné sur une cornée, c'est à dire que la membrane amniotique (2) épouse la forme de la cornée.

Dans un mode de réalisation, la membrane amniotique (2) selon invention, la membrane amniotique indépendante selon invention, la membrane amniotique centrale selon invention et/ou la structure annulaire selon invention sont stériles.

Dans un mode de réalisation, le dispositif utilisable comme lentille selon l'invention est stérile.

Selon la présente invention, on entend par « stérile » une structure exempte de germe, à l'état naturel ou après stérilisation.

La stérilisation pourra être effectuée par toute les méthodes classiquement utilisées par l'Homme du métier.

Dans un mode de réalisation, la stérilisation sera réalisée en irradiation gamma.

Les membranes amniotiques étant des tissus biologiques, le risque de transmission d'une infection d'un donneur vers un receveur, ou de contamination lors de la collecte du tissu à l'accouchement, lors des traitements préparatoires du tissu, ou lors du transport de ce tissu est important. De fait, un procédé efficace tant de désinfection que de conservation de ces tissus est vital pour la sécurité des patients.

Dans un mode de réalisation, la membrane amniotique (2) selon invention, la membrane amniotique indépendante selon invention, la membrane amniotique centrale selon invention et/ou la structure annulaire selon invention sont viro-inactivées.

Dans un mode de réalisation, le dispositif utilisable comme lentille selon l'invention est viro-inactivé.

Par « viro-inactivation », on entend une technique qui permet de réduire fortement ou totalement, et définitivement, la capacité d'action des virus. Ceux-ci, définis comme inactivés, perdent leurs capacités pathogéniques et de réplication par une diminution de leur population de 4 log lors des titrages résiduels qui suivent une ou deux étapes chimiques indépendantes, que ce soit sur des virus enveloppés, ou non enveloppés, ADN ou ARN.

Un tel procédé est par exemple décrit dans le document WO2017/140914 au nom de TBF GENIE TISSULAIRE.

Dans un mode de réalisation, le dispositif utilisable comme lentille selon l'invention est caractérisé en ce que la membrane amniotique (2) est viro-inactivée selon les deux étapes de viro-inactivation chimiques du procédé décrit dans WO2017/140914.

Dans un mode de réalisation, le dispositif utilisable comme lentille selon l'invention est caractérisé en ce que la membrane amniotique (2) selon invention, la membrane amniotique indépendante selon invention, la membrane amniotique centrale selon invention et/ou la structure annulaire selon invention est obtenue selon le procédé décrit dans WO2017/140914.

Dans un mode de réalisation, le dispositif utilisable comme lentille selon l'invention est caractérisé en ce que la membrane amniotique centrale est constituée de deux fragments de membrane amniotique superposés de façon à ce que les faces épithéliales de chacun des fragments soient sur les faces externes de la membrane amniotique centrale double épaisseur réalisée par la superposition.

La membrane amniotique centrale en double épaisseur est réalisée de façon à ce que, pour chaque épaisseur de membrane amniotique centrale, l'épithélium soit orienté de façon à être en contact avec l'oeil pour une épaisseur, et en contact avec l'intérieur des paupières pour l'autre épaisseur. Une telle configuration de membrane amniotique centrale est particulièrement utile dans le traitement du syndrome de Stevens-Johnson ou du syndrome de Lyell.

Lorsque la structure annulaire selon l'invention est circulaire elle est définie par son diamètre, lorsqu'elle est de forme elliptique ou ovale elle sera définie par un diamètre équivalent.

Dans un mode de réalisation, le dispositif utilisable comme lentille selon l'invention est caractérisé en ce que la structure annulaire selon l'invention possède un diamètre compris entre 1 et 3 cm.

Dans un mode de réalisation, le dispositif utilisable comme lentille selon l'invention est caractérisé en ce que la structure annulaire selon l'invention possède un diamètre compris entre 2 et 2,5 cm.

Lorsque le dispositif utilisable comme lentille selon l'invention se présente sous forme de disque, le diamètre du disque est compris entre 2 cm et 2,5 cm.

Dans un mode de réalisation, la structure annulaire selon l'invention, la structure cylindrique selon l'invention, la membrane amniotique (2) selon l'invention, la membrane amniotique centrale selon l'invention, la membrane amniotique indépendante selon l'invention et/ou le dispositif utilisable comme lentille selon l'invention sont caractérisés en ce qu'ils sont décellularisées.

La décellularisation pourra être effectuée par toute les méthodes classiquement utilisées par l'Homme du métier.

Dans un mode de réalisation, la décellularisation est effectuée par incubation dans de l'acide éthylène diamine tétra acétique (EDTA).

Dans un mode de réalisation, le dispositif utilisable comme lentille selon l'invention est lyophilisé.

Dans un mode de réalisation, le dispositif utilisable comme lentille selon l'invention comprend en outre de gelée de Wharton à la surface de la membrane amniotique centrale.

Dans un mode de réalisation, la gelée de Wharton est imprégnée d'au moins un principe actif.

Dans un mode de réalisation, la gelée de Wharton est imprégnée d'une solution comprenant des exosomes.

Dans un mode de réalisation, la membrane amniotique (2) selon invention, la membrane amniotique indépendante selon invention, la membrane amniotique centrale selon invention, la structure annulaire selon invention et/ou le dispositif utilisable comme lentille selon l'invention sont imprégnées d'au moins un principe actif.

Dans un mode de réalisation, la membrane amniotique (2) selon invention, la membrane amniotique indépendante selon invention, la membrane amniotique centrale selon invention, la structure annulaire selon invention et/ou le dispositif utilisable comme lentille selon l'invention sont imprégnées d'une solution comprenant des exosomes.

Dans un mode de réalisation, le principe actif imprégné est choisi dans le groupe constitué des antibiotiques, des antiseptiques, des antiviraux, des anticorps monoclonaux, des inhibiteurs semi-synthétiques des métalloprotéases, des agents immunosuppresseurs, des anti-inflammatoires, des antifongiques, des anti-allergiques, des anesthésiques, ou des protéines immunoadhésives, des agents permettant d'éviter les sécheresses oculaires, seuls ou en combinaisons.

Le au moins le principe actif imprégné peut être un principe actif qui est naturellement présent ou non dans la gelée de Wharton, la membrane amniotique (2) selon invention, la membrane amniotique indépendante selon invention, la membrane amniotique centrale selon invention, la structure annulaire selon invention et/ou le dispositif utilisable comme lentille selon l'invention.

Dans un mode de réalisation, le principe actif imprégné est choisi dans le groupe constitué des antibiotiques. Quelques exemples d'antibiotiques utilisables sont donnés ci-après : tétracyclines (daunomycine, tétracycline, chloretetracycline, oxytétracycline, etc.), glycopeptides (vancomycine, etc.), aminoglycosides (gentamycine, etc.), aminosides (tobramycine, néomycine, etc.), fluoroquinolones (ciprofloxacine, moxifloxacin, etc.), quinolones (gatifloxacine, etc.), polypeptides (bacitracine, polymyxine, etc.), phénicolés (chloramphénicol, etc.), macrolides (erythromycine, etc.), sulfonamides (sulfacétamide, sulfaméthoxazole, sulfisoxazole, etc.), céphalosporines (céfradoxil, céfoxitine, etc.), et tout autre antibiotique.

Dans un mode de réalisation, le principe actif imprégné est choisi dans le groupe constitué des anti-inflammatoires stéroïdiens (AIS) et/ou non-stéroïdiens (AINS). Quelques exemples d'AIS sont donnés ci-après : triamcinolone, dexaméthasone, prednisolone, hydrocortisone, corticostérone, fluocinolone, prednisolone, méthylprednisolone, fluorométholone, betaméthasone, tétrahydrocortisol, riméxolone, etc. Quelques exemples d'AINS sont donnés ci-après : indométacine, népafénac, diclofénac, bromfénac, kétorolac, suprofène, etc.

Dans un mode de réalisation, le principe actif imprégné est choisi dans le groupe constitué des immunosuppresseurs, une liste non-limitative est donnée ci-après : déxaméthasone, bétaméthasone, etc.

Dans un mode de réalisation, le principe actif imprégné est choisi dans le groupe constitué des antiviraux, une liste non-limitative est donnée ci-après : ganciclovir, trifluorothymidine, aciclovir, DDI, AZT, foscarnet, vidarabine, trifluorouridine, idoxuridine, ribavirine, inhibiteurs de protéase, agent anticytomégalovirus, etc.

Dans un mode de réalisation, le principe actif imprégné est choisi dans le groupe constitué des antifongiques, une liste non-limitative est donnée ci-après : fluconazole, nitrofurazone, amphotéricine B, kétoconazole, etc.

Dans un mode de réalisation, le principe actif imprégné est choisi dans le groupe constitué des antiallergiques, une liste non-limitative est donnée ci-après : méthapyriline, chlorpheniramine, pyrilamine, prophenpyridamine, etc.

Dans un mode de réalisation, le principe actif imprégné est choisi dans le groupe constitué des anesthésiques, une liste non-limitative est donnée ci-après : lidocaïne, mépivacaïne, etc.

Dans un mode de réalisation, le principe actif imprégné est choisi dans le groupe constitué des agents permettant d'éviter les sécheresses oculaires, une liste non-limitative est donnée ci-après : azithromycine, ciclosporine, lubrifiants etc...

Dans un mode de réalisation, l'imprégnation est réalisée selon le protocole décrit dans la demande FR2107114 ou PCT/EP2022/067938.

Dans un mode de réalisation, l'imprégnation est précédée de la lyophilisation de la gelée de Wharton, la membrane amniotique (2) selon invention, la membrane amniotique indépendante selon invention, la membrane amniotique centrale selon invention, la structure annulaire selon invention et/ou du dispositif utilisable comme lentille selon l'invention suivi de la mise en contact avec une solution comprenant des exosomes et/ou le principe actif.

Dans un mode de réalisation, la mise en contact est réalisée par dépôt de la solution comprenant les exosomes et/ou le principe actif à la surface de la gelée de Wharton lyophilisée, la membrane amniotique (2) selon invention lyophilisée, la membrane amniotique indépendante selon invention lyophilisée, la membrane amniotique centrale selon invention lyophilisée, la structure annulaire selon invention lyophilisée et/ou le dispositif utilisable comme lentille selon l'invention lyophilisé.

Au sens de la présente invention, par « imprégnation » et/ou « imprégné » on entend que la solution comprenant des exosomes et/ou le principe actif pénètre dans la gelée de Wharton lyophilisée, la membrane amniotique (2) selon invention lyophilisée, la membrane amniotique indépendante selon invention lyophilisée, la membrane amniotique centrale selon invention lyophilisée, la structure annulaire selon invention lyophilisée et/ou le dispositif utilisable comme lentille selon l'invention lyophilisé et s'y répand, s'y diffuse.

L'imprégnation consiste donc à faire entrer la solution comprenant les exosomes et/ou le principe actif dans la gelée de Wharton lyophilisée, la membrane amniotique (2) selon invention lyophilisée, la membrane amniotique indépendante selon invention lyophilisée, la membrane amniotique centrale selon invention lyophilisée, la structure annulaire selon invention lyophilisée et/ou le dispositif utilisable comme lentille selon l'invention lyophilisé qui sont alors au moins partiellement réhydraté et les exosomes et/ou le principe actif sont enchâssés dans ceux-ci de sorte qu'ils puissent être à nouveau lyophilisés pour être conservés sans perdre leurs contenus en exosomes et/ou en principe actif.

Ainsi, l'imprégnation selon l'invention permet d'ajouter une quantité définie d'exosomes et/ou de principe actif selon l'invention qui n'était pas présente dans la gelée de Wharton, la membrane amniotique (2) selon invention, la membrane amniotique indépendante selon invention, la membrane amniotique centrale selon invention, la structure annulaire selon invention et/ou le dispositif utilisable comme lentille selon l'invention, avant imprégnation.

Dans un mode de réalisation, les exosomes sont isolés par centrifugation, filtration, ultrafiltration et/ou l'immunoprécipitation du milieu de culture des cellules d'i ntérêt.

Dans un mode de réalisation, les cellules d'intérêt sont choisies dans le groupe consistant en les macrophages, plaquettes sanguines, cellules dendritiques, cellules souches mésenchymateuses, cellules souches pluripotentes induites, de cellules de la moelle osseuse, du tissu adipeux et/ou de cordon ombilical et/ou sont purifiées à partir de fluides biologiques.

Dans un mode de réalisation, lesdits exosomes sont issus de cellules souches mésenchymateuses.

Dans un mode de réalisation, lesdits exosomes sont issus de cellules souches mésenchymateuses humaines.

Dans un mode de réalisation, lesdites cellules souches mésenchymateuses sont issues de cordon ombilical.

Dans un mode de réalisation, les cellules souches mésenchymateuses sont issues de cordon ombilical humain.

Dans un mode de réalisation, la collecte des exosomes dans le milieu de culture des cellules d'intérêt est effectuée par centrifugation.

Dans un mode de réalisation, la solution comprenant des exosomes est une solution aqueuse.

Dans un mode de réalisation, la solution comprenant des exosomes comprend en outre le milieu de culture des cellules d'intérêt duquel les exosomes ont été extraits.

Dans un mode de réalisation, la solution comprenant des exosomes comprend en outre du tampon phosphate salin.

Dans un mode de réalisation, la solution comprenant des exosomes comprend en outre un autre principe actif.

Un autre objet de la présente invention concerne un dispositif de d'administration d'exosomes comprenant la structure annulaire selon invention ou le dispositif utilisable comme lentille selon l'invention, imprégné d'une solution comprenant des exosomes selon l'invention.

Dans un mode de réalisation, le dispositif d'administration selon l'invention consiste en une structure annulaire selon invention ou un dispositif utilisable comme lentille selon l'invention, imprégné d'une solution comprenant des exosomes selon l'invention.

Dans un mode de réalisation, le dispositif d'administration selon l'invention est un dispositif d'administration à libération prolongée d'exosomes et/ou de principe actif.

Dans un mode de réalisation, le dispositif d'administration à libération prolongée d'exosomes selon l'invention permet une libération des exosomes et/ou du principe actif pendant au moins 4 heures à compter de l'administration.

Dans un mode de réalisation, le dispositif d'administration à libération prolongée d'exosomes selon l'invention permet une libération des exosomes et/ou du principe actif pendant au moins 24 heures à compter de l'administration.

Dans un mode de réalisation, le dispositif d'administration à libération prolongée d'exosomes selon l'invention permet une libération des exosomes et/ou du principe actif pendant une durée de 24 à 72 heures à compter l'administration.

Dans un mode de réalisation, le dispositif d'administration à libération prolongée d'exosomes selon l'invention permet une libération des exosomes et/ou du principe actif pendant au moins 72 heures à compter de l'administration.

Dans un mode de réalisation, le dispositif d'administration à libération prolongée d'exosomes selon l'invention permet une libération des exosomes et/ou du principe actif pendant une durée de 4 heures à 1 semaine à compter de l'administration.

La présente invention concerne également un procédé de fabrication d'une structure annulaire constituée de membrane amniotique (2) comprenant les étapes suivantes :
a) on dispose d'une membrane amniotique (2) en forme de disque ou d'anneau,
b) on dispose d'un support de type filaire (1),
c) on procède à une étape d'enroulement de la membrane amniotique (2) autour du support de type filaire (1) à la périphérie du disque ou de l'anneau,
d) on procède à une étape de réticulation,
e) on obtient une structure annulaire à la périphérie du disque de membrane amniotique (2) ou de forme annulaire.

La présente invention concerne également un procédé de fabrication d'un dispositif utilisable comme lentille selon l'invention comprenant les étapes suivantes :
a) on dispose d'une membrane amniotique (2) en forme de disque,
b) on dispose d'un support de type filaire (1),
c) on procède à une étape d'enroulement de la membrane amniotique (2) autour du support de type filaire (1) à la périphérie du disque,
d) on procède à une étape de réticulation,
e) on obtient un dispositif utilisable comme lentille selon l'invention.

Dans un mode de réalisation du procédé de fabrication d'un dispositif utilisable comme lentille selon l'invention, la réticulation selon l'étape d) est réalisée uniquement sur la structure annulaire selon l'invention, la membrane amniotique centrale du dispositif selon l'invention ne subit pas d'étape de réticulation.

Dans un mode de réalisation, le procédé de fabrication d'un dispositif utilisable comme lentille selon l'invention comprend en outre l'ajout de gelée de Wharton à la surface de la membrane amniotique centrale.

Dans un mode de réalisation, la gelée de Wharton est imprégnée d'au moins un principe actif.

Dans un mode de réalisation, la gelée de Wharton est imprégnée d'une solution d'exosomes.

Dans un mode de réalisation, l'imprégnation est réalisée par une étape préalable de lyophilisation de la gelée de Wharton.

Dans un mode de réalisation, l'imprégnation de la gelée de Wharton est réalisée selon le protocole décrit dans la demande FR2107114 ou PCT/EP2022/067938.

Dans un mode de réalisation, l'un quelconque des procédés selon la présente invention ne comprend pas de mise en contact de la membrane amniotique avec un gel durcissable à la périphérie de celle-ci et/ou avec la structure annulaire.

La présente invention concerne également un procédé de fabrication d'une structure cylindrique selon l'invention constituée de membrane amniotique (2) comprenant les étapes suivantes :
a) on dispose d'une membrane amniotique (2) en forme de rectangle,
b) on dispose d'un support de type filaire (1),
c) on procède à une étape d'enroulement de la membrane amniotique (2) autour du support de type filaire (1),
d) on procède à une étape de réticulation,
e) on obtient une structure cylindrique constituée de membrane amniotique (2) ayant subi une étape de réticulation.

Dans un mode de réalisation, le procédé de fabrication d'une structure cylindrique selon l'invention comprend une étape de retrait du support de type filaire (1) après l'étape d) de réticulation.

Dans un mode de réalisation, le procédé de fabrication d'une structure cylindrique selon l'invention comprend une étape de suture (3) de la structure cylindrique pour former un anneau.

La présente invention concerne également un procédé de fabrication d'une structure annulaire selon l'invention constituée de membrane amniotique (2), comprenant les étapes suivantes :
a) on dispose d'une membrane amniotique (2) en forme de rectangle,
b) on dispose d'un support de type filaire (1),
c) on procède à une étape d'enroulement de la membrane amniotique (2) autour du support de type filaire (1),
d) on procède à une étape de réticulation,
d') on retire le support de type filaire (1),
e) on obtient une structure cylindrique,
e') on procède à une suture (3) des extrémités de la structure cylindrique pour former un anneau.

Dans un mode de réalisation de l'un quelconque des procédés selon l'invention, la structure annulaire, la structure cylindrique et/ou le dispositif formés à l'issu du procédé comprennent le support de type filaire (1).

Dans un mode de réalisation de l'un quelconque des procédés selon l'invention, le procédé comprend une étape de retrait du support de type filaire (1) après l'étape d) de réticulation.

Dans un mode de réalisation de l'un quelconque des procédés selon l'invention comprend en outre une étape de lyophilisation de la structure obtenue.

Au sens de la présente invention, on entend par « lyophilisation », une technique visant à dessécher un produit préalablement surgelé par sublimation. Plus précisément, le liquide à ôter du produit est dans un premier temps transformé en glace par congélation ; puis par une dessiccation primaire, sous vide, la glace est sublimée ; enfin par une dessiccation secondaire, les molécules d'eau à la surface du produit sont extraites par désorption.

La lyophilisation pourra être effectuée par toute les méthodes classiquement utilisées par l'Homme du métier.

Dans un mode de réalisation, la lyophilisation sera réalisée par un procédé tel que décrit dans le document WO2017/140914 au nom de TBF GENIE TISSULAIRE.

Dans un mode de réalisation, l'étape de lyophilisation est suivie d'une étape d'imprégnation de la structure obtenue, telle que définie ci-dessus, d'une solution comprenant des exosomes et/ou un principe actif.

Dans un mode de réalisation, l'étape d'imprégnation est réalisée selon le protocole décrit dans la demande FR2107114 ou PCT/EP2022/067938.

Dans un mode de réalisation, l'un quelconque des procédés selon l'invention comprend en outre une étape de réhydratation avant utilisation.

Dans un mode de réalisation, l'un quelconque des procédés selon l'invention comprend une étape de séchage de la membrane amniotique (2) avant l'étape d'enroulement.

Dans un mode de réalisation de l'un quelconque des procédés selon l'invention, l'étape d) de réticulation est effectuée par réticulation par rayonnement UV de la membrane en présence de riboflavine ou d'un de ses sels.

Dans mode de réalisation, l'étape de réticulation est effectuée par réticulation par rayonnement UV de la membrane en présence de sel de sodium phosphaté de riboflavine.

Dans un mode de réalisation, la réticulation est réalisée dans un moule transparent à la lumière UV et comportant une rigole dans laquelle est introduite l'agent photosensibilisateur.

Dans un mode de réalisation, lorsque la membrane amniotique est enroulée sur elle-même, et éventuellement autour d'un fil, l'étape de réticulation comprend la réticulation du collagène d'une épaisseur de l'enroulement de membrane amniotique avec le collagène d'au moins une autre épaisseur de l'enroulement de membrane amniotique.

La riboflavine et de façon préférentielle le sel de sodium phosphaté de riboflavine est une molécule photosensibilisante et photopolymérisante qui a une capacité de diffusion faible. La riboflavine est un constituant essentiel des cellules vivantes et est non cytotoxique, son utilisation sera donc sans aucune conséquence sur la toxicité des implants ainsi fabriqués.

Un autre objet de la présente invention concerne le dispositif utilisable comme lentille selon l'invention pour son utilisation thérapeutique.

Un autre objet de la présente invention concerne le dispositif utilisable comme lentille selon l'invention pour son utilisation dans le traitement des lésions oculaires et/ou des sécheresses oculaires.

Dans un mode de réalisation, le dispositif utilisable comme lentille selon l'invention pour son utilisation dans le traitement des maladies inflammatoires de la conjonctive et/ou de la cornée.

Dans un mode de réalisation, le dispositif utilisable comme lentille selon l'invention pour son utilisation dans le traitement des infections de la conjonctive et/ou cornée.

Dans un mode de réalisation, le dispositif utilisable comme lentille selon l'invention pour son utilisation le traitement des destructions de la surface de la conjonctive et/ou cornée.

Dans un mode de réalisation, le dispositif utilisable comme lentille selon l'invention est destiné à être utilisé dans le traitement de lésions de la conjonctive et cornée choisies dans le groupe constitué des ulcères cornéens, des brûlures, du syndrome de Stevens-Johnson, du syndrome de Lyell et/ou les kérato-conjonctivites immunitaires, allergiques, inflammatoire et/ou traumatiques.

Un autre objet de la présente invention concerne une structure annulaire selon l'invention pour son utilisation thérapeutique.

Un autre objet de la présente invention concerne une structure cylindrique selon l'invention pour son utilisation thérapeutique.

Un autre objet de la présente invention concerne une méthode de traitement caractérisé en ce qu'elle comprend l'application sur la partie du corps à traiter, d'un dispositif selon l'invention.

Un autre objet de la présente invention concerne une méthode de traitement thérapeutique des lésions oculaires et/ou des sécheresses oculaires et/ou des maladies inflammatoires de la conjonctive et/ou de la cornée et/ou des infections de la conjonctive et/ou cornée et/ou des destructions de la surface de la conjonctive et/ou cornée des et/ou ulcères cornéens, des brûlures, du syndrome de Stevens-Johnson, du syndrome de Lyell et/ou les kérato-conjonctivites immunitaires, allergiques, inflammatoire et/ou traumatiques caractérisé en ce qu'elle comprend l'application sur la partie de l'oeil à traiter d'un dispositif selon l'invention.

Dans les exemples, tous les pourcentages sont donnés en poids, sauf indication contraire, la température est exprimée en degré Celsius sauf indication contraire, et la pression est la pression atmosphérique, sauf indication contraire.

### Exemple 1 : Préparation d'une membrane amniotique viro-inactivée selon l'invention :

Un donneur proprement informé et consentant en accord avec les exigences de la Déclaration d'Helsinki offre en don le tissu placentaire issu d'un accouchement. Par exigence sanitaire relative aux dons de tissus et de cellules d'origine humaine, une qualification en amont du donneur est systématique. Cette qualification implique une recherche de virus HIV, hépatites B, C, HTLV et de la bactérie tréponème pâle responsable de la syphilis.

Le tissu placentaire est récupéré le plus tôt possible en salle d'accouchement suite à un accouchement. Il est placé dans une boîte stérile puis congelé à une température de -20°C.

Ce tissu placentaire isolé est conservé à sec à une température de -80°C jusqu'à une durée de deux ans ou être directement traité.

Au laboratoire, en salle stérile, la procédure suivante est appliquée :
La membrane amniotique est isolée du placenta et est nettoyée.

La membrane amniotique subit une succession de bains assurant un traitement chimique de celle-ci. Ce traitement a pour objectif une désinfection de la membrane amniotique, et tout particulièrement sa viro-inactivation. Une agitation douce à environ 30 rpm du milieu liquide est appliquée lors de chaque bain afin d'assurer une pénétration homogène des solvants dans les tissus.

Dans un premier temps, la membrane amniotique est déposée dans un bain d'eau purifiée à température ambiante pendant environ 3 heures. Cette étape assure tant la décongélation du tissu physiologique qu'une première étape de lyse cellulaire par pression osmotique.

Puis, la membrane amniotique est transférée dans un bain décontaminant composé d'éthanol 70% v/v en volume d'éthanol par rapport au volume total de la solution à température ambiante pendant environ 1 heure.

Un lavage est effectué dans de l'eau purifiée pendant environ 15 minutes à température ambiante pour retirer l'éthanol.

Afin d'assurer la seconde étape de traitement décontaminant, la membrane amniotique est transférée dans un bain composé de peroxyde d'hydrogène à 30% p/v en poids de peroxyde d'hydrogène par rapport au volume total de la solution à température ambiante pendant environ 15 minutes.

Puis la membrane amniotique est transférée dans un bain décontaminant composé de peroxyde d'hydrogène à 3% p/v en poids de peroxyde d'hydrogène par rapport au volume total de la solution à température ambiante pendant environ 1 heure.

L'action chimique appliquée à la membrane amniotique est neutralisée dans deux bains successifs comportant de l'hydroxyde de sodium dilué à un pH de 8,5. Les bains de neutralisation s'effectuent à température ambiante pendant environ 15 minutes.

Afin d'assurer un rééquilibrage du pH et pour éliminer au mieux les résidus organiques se détachant du tissu d'intérêt, la membrane amniotique est transférée dans deux bains successifs de tampon phosphate salin afin d'assurer son rééquilibrage physiologique. Les bains de tampon phosphate salin s'effectuent à température ambiante pendant environ 15 minutes.

Enfin, la membrane amniotique (2) est transférée dans un dernier bain à l'eau purifiée, à température ambiante, pendant au moins 15 minutes et jusqu'à environ 1 heure.

On obtient une membrane amniotique désinfectée viro-inactivée et décellularisée.

### Exemple 2 : Fabrication des dispositifs utilisables comme lentille selon l'invention

### Exemple 2.1 : Structure annulaire à la périphérie d'un disque constitué de membrane amniotique (2) selon l'invention.

La membrane amniotique (2) telle qu'obtenue à l'exemple 1 est découpée en forme de disque, puis enroulée sur sa périphérie sur le support de type filaire (1) qui est un fil de suture en nylon monofilament.

L'étape de réticulation est effectuée uniquement sur la partie périphérique et donc uniquement sur la partie enroulée de la membrane amniotique (2).

Cette étape est effectuée en utilisant un moule transparent à la lumière UV et comportant une rigole dans laquelle est introduite une solution de riboflavine à une concentration de 0.2 g/L.

La membrane amniotique (2) est disposée dans le moule de sorte que seule la partie périphérique et donc uniquement sur la partie enroulée de la membrane amniotique (2) repose dans la rigole contenant la riboflavine.

Le moule contenant la solution de riboflavine et le disque de membrane amniotique (2) enroulé autour du support de type filaire (1) est ensuite soumis à un rayonnement UV de 14,4 joules pendant 30 minutes.

Le disque obtenu correspond à un dispositif selon l'invention comprenant une membrane amniotique centrale et une structure annulaire selon l'invention, solidaire de ladite membrane amniotique centrale et disposée à la périphérie de celle-ci.

Le disque obtenu peut être lyophilisé et conservé.

Lors de la réhydratation au moment de l'utilisation, par ajout de sérum physiologique, la membrane amniotique (2) ne va pas se dérouler et la forme d'anneau sera conservée.

Ce procédé et la lentille obtenue permettent cette conservation de la forme annulaire sans montage compliqué ni apport de matériau supplémentaire.

Dans une variante, le fil de suture est retiré après l'étape de réticulation.

Le dispositif obtenu peut être utilisé comme lentille cornéenne.

### Exemple 2.2 : Structure cylindrique constituée de membrane amniotique (2) selon l'invention.

La membrane amniotique (2) est découpée en forme de rectangle, puis enroulée complètement sur le support de type filaire (1) qui peut être un fil de suture en nylon monofilament pour obtenir un cylindre constitué de membrane amniotique (2) roulée sur un fil de suture.

L'étape de réticulation est effectuée sur la totalité du cylindre.

Cette étape est effectuée en utilisant un moule transparent à la lumière UV et comportant une rigole dans laquelle est introduite une solution de riboflavine à une concentration de 0.2 g/L.

Le moule contenant la solution de riboflavine et le cylindre constitué de membrane amniotique (2) enroulée autour du support de type filaire (1) est ensuite soumis à un rayonnement UV de 14,4 joules pendant 30 minutes. Pour parfaire la réticulation une répétition de cette étape peut être effectuée.

On obtient un cylindre selon l'invention constituée de membrane amniotique (2) ayant subi une étape de réticulation.

Le cylindre obtenu est lyophilisé et conservé.

Dans une variante le fil de suture est retiré après réticulation.

Dans une variante le fil est retiré après réticulation et les extrémités du cylindre sont suturées pour obtenir une forme annulaire.

### Exemple 2.3 : Structure annulaire constituée de membrane amniotique (2) sans suture selon l'invention.

Une membrane amniotique (2) est découpée en forme d'anneau, puis enroulée complètement sur le support de type filaire (1) qui est un fil de suture en nylon monofilament, pour obtenir une structure annulaire constituée de membrane amniotique (2) roulée sur un fil de suture.

Après enroulement, les autres étapes du procédé sont identiques à celles-ci-dessus décrites à l'exemple 2b).

On obtient un dispositif de forme annulaire.

## Revendications

1. Structure annulaire constituée de membrane amniotique (2) ayant subi une étape de réticulation.

2. Structure annulaire selon la revendication 1, dans laquelle la membrane amniotique (2) est enroulée sur elle-même et/ou autour d'un support de type filaire (1).

3. Structure annulaire l'une quelconque des revendications 1 ou 2, dans laquelle l'étape de réticulation est réalisée par glycation non enzymatique, irradiation à la lumière UV avec ou sans un agent photosensibilisateur et/ou par réaction aldéhydique.

4. Procédé de fabrication d'une structure annulaire constituée de membrane amniotique (2) selon l'une quelconque des revendications 1 à 3, comprenant les étapes suivantes :
a) on dispose d'une membrane amniotique (2) en forme de disque,
b) on dispose d'un support de type filaire (1),
c) on procède à une étape d'enroulement de la membrane amniotique (2) autour du support de type filaire (1) à la périphérie du disque,
d) on procède à une étape de réticulation,
e) on obtient une structure annulaire à la périphérie du disque de membrane amniotique (2).

5. Procédé de fabrication d'une structure annulaire constituée de membrane amniotique (2) selon l'une quelconque des revendications 1 à 3, comprenant les étapes suivantes :
a) on dispose d'une membrane amniotique (2) en forme de rectangle,
b) on dispose d'un support de type filaire (1),
c) on procède à une étape d'enroulement de la membrane amniotique (2) autour du support de type filaire (1),
d) on procède à une étape de réticulation,
d') on retire le support filaire (1),
e) on obtient une structure cylindrique.
e') on procède à une suture (3) des extrémités de la structure cylindrique pour former un anneau.

6. Procédé selon la revendication 4, **caractérisé en ce qu'**il comprend une étape de retrait du support de type filaire (1) après l'étape de réticulation.

7. Procédé selon l'une quelconque des revendications 4 à 6, **caractérisé en ce qu'**il comprend en outre une étape de lyophilisation après l'obtention de la structure attendue.

8. Procédé selon l'une quelconque des revendications 4 à 7, **caractérisé en ce qu'**il comprend en outre une étape de réhydratation avant utilisation.

9. Procédé selon l'une quelconque des revendications 4 à 8, caractérisé en ce l'étape de réticulation est effectuée par réticulation par rayonnement UV de la membrane amniotique (2) en présence de riboflavine.

10. Dispositif, utilisable comme lentille, comprenant :
- une membrane amniotique centrale ;
- une structure annulaire selon l'une quelconque des revendications 1 à 3, solidaire de ladite membrane centrale et disposée à la périphérie de celle-ci.

11. Dispositif selon la revendication 10, dans lequel la membrane amniotique centrale du dispositif n'est pas réticulée, seule la structure annulaire est réticulée.

12. Procédé de fabrication d'un dispositif selon l'une quelconque des revendications 10 ou 11, comprenant les étapes suivantes :
a) on dispose d'une membrane amniotique (2) en forme de disque,
b) on dispose d'un support de type filaire (1),
c) on procède à une étape d'enroulement de la membrane amniotique (2) autour du support de type filaire (1) à la périphérie du disque,
d) on procède à une étape de réticulation.

13. Procédé de fabrication d'un dispositif selon la revendication 12, dans lequel la réticulation selon l'étape d) est réalisée uniquement sur la structure annulaire, la membrane amniotique centrale du dispositif ne subissant pas d'étape de réticulation.
